(19) Europäisches Patentamt
European Patent Office
Office européen des brevets

(11) Publication number: 0 322 630 B1

(12) # EUROPEAN PATENT SPECIFICATION

(45) Date of publication of patent specification: 06.10.93

(51) Int. Cl.5: C12P 33/08, C07J 71/00, C12P 33/02

(21) Application number: 88120781.5

(22) Date of filing: 13.12.88

(54) **Process for the preparation of pregneno-oxazolines.**

(30) Priority: 29.12.87 GB 8730216

(43) Date of publication of application: 05.07.89 Bulletin 89/27

(45) Publication of the grant of the patent: 06.10.93 Bulletin 93/40

(84) Designated Contracting States: AT BE CH DE ES FR GB GR IT LI LU NL SE

(56) References cited:
EP-A- 0 055 832
CH-A- 484 887
GB-A- 1 077 393
US-A- 3 452 005

ATCC's Catalogue of Strains I, 14th edition, 1980, pages 34 and 292

ATCC's Catalogue of Filamentous Fungi, 18th ed., 1991, page 139

ATCC's Catalogue of Bacteria and Bacteriophages, 18th ed., 1992, pages 31 and 227

(73) Proprietor: GRUPPO LEPETIT S.p.A.
Via G. Murat 23
I-20159 Milano(IT)

(72) Inventor: Assi, Francesco
34, Via Corridoni
I-20063 Cernusco sul Naviglio (MI)(IT)

(74) Representative: Sgarbi, Renato et al
GRUPPO LEPETIT S.p.A.
Patent and Trademark Department
Via Roberto Lepetit, 34
I-21040 Gerenzano (Varese) (IT)

EP 0 322 630 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to a process for the preparation of pregneno-oxazolines of formula I

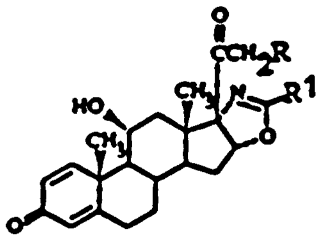

I

wherein:

$R^1$ represents hydrogen, ($C_1$-$C_8$)alkyl or phenyl and

R represents hydrogen or hydroxy.

Pregneno-oxazolines having the above formula and in particular the 21-acyl or hydroxy acyl derivatives thereof are described in GB-B- 1077393. They are reported to have anti-inflammatory, glucocorticoid and hormone-like pharmacological activity.

A member of this class, 11-beta-hydroxy-2'-methyl-3,20-dioxo-5'-beta-H-1,4-pregnadieno-[16α,17α-d]-oxazoline-21-acetate, Deflazacort (INN), was recently introduced in therapy as a calcium-sparing glucocorticoid agent.

A 21-hemisuccinate derivative of the compounds of formula I is described in GB-A- 2075511.

GB-B- 1077393 describes a chemical semi-synthetic process for the preparation of the above compounds, while GB-B- 1205026 describes a microbiological process for preparing DELTA-4,5-11-beta-hydroxy-steroido-oxazolines from DELTA-4,5-steroido-oxazolines in the presence of a fungus selected from Fungi imperfecti and Phycomycetes. Among the strains cited therein, there is Curvularia lunata NRRL 2380.

EP-B- 55832 describes the preparation of 11-beta-21-dihydroxy-2′-methyl-5′-beta-H-1,4-pregnadieno-(16,17-d)-oxazolyl-3,20-dione from the fermentation of 11-beta-21-dihydroxy-2′-methyl-5′-beta-H-4-pregneno (16,17-d)oxazolyl-3,20-dione in a living culture of Arthrobacter simplex in the presence of 0.04 to 0.12g cobalt (II) ions per liter of culture.

According to the method of the invention, a 2′-substituted-4-pregnen-21-ol-[17alpha,16alpha-d-]-oxazolin yl-3,20-dione of formula II

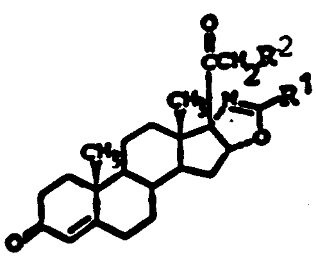

II

wherein

$R^2$ represents hydrogen or acyloxy and $R^1$ is as defined above, is contacted sequentially with a growing mixed culture of a Curvularia strain and an Arthrobacter strain to transform it into a compound of formula I.

Preferably acyloxy is ($C_2$-$C_4$)acyloxy and most preferably acetyloxy ($CH_3COO$).

The biological transformation of the compounds of formula II is obtained in two subsequent steps conveniently without isolating the intermediate.

The Curvularia strain is preferably Curvularia lunata NRRL 2380 while the Arthrobacter strain is preferably Arthrobacter simplex ATCC 6946.

The intermediate of the following formula III

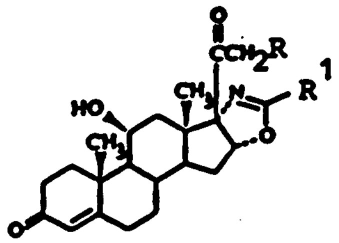

III

wherein R and R[1] are defined as above, which forms, is never isolated, according to a preferred practice of the invention, but is transformed in situ into a compound of formula I.

The compounds of formula I can be in turn transformed into the known 21-hydroxy, 21-acyl, hydroxy-acyl or carboxy-acyl derivatives by techniques known per se in the art (see for instance GB-B- 1077393 or GB-A- 2075511).

Apart from their own biological activity, the compounds of formula I are mainly useful as intermediates in the preparation of the above mentioned derivatives such as Deflazacort or Deflazacort hemisuccinate.

More particularly, a compound of formula II, and preferably a compound of formula II wherein R represents hydrogen, is added to a growing culture of C. lunata (preferably C. lunata NRRL 2380) in a suitable medium containing assimilable sources of carbon, nitrogen and inorganic salts, after 12-24 h from inoculum, and, after 36-96 h from inoculum, and preferably 48-72 h after inoculum, a growing culture of A. simplex (preferably A. simplex ATCC 6946) of 12-36 h, preferably of 18-36 h, is added to this mixture and further cultivated.

Harvesting is generally started 72-144 h after the beginning of the fermentation of C. lunata.

The fermentation is conducted under submerged aerobic conditions; the air-flow, agitation, foaming etc. can be controlled as known in the art.

The temperature is generally kept between 27 °C and 35 °C and the pH between 6.0 and 7.5.

The fermentation medium is one which has assimilable sources of carbon, nitrogen and inorganic salts. Suitable fermentation media are generally known and available to the man skilled in the art and include the usual media for the cultivation of Curvularia or Arthrobacter strains. Preferred examples of nitrogen sources for these media are soybean meal, yeast extract, corn steep liquor (CSL), casein and peptone; preferred sources of carbon are dextroses, disaccharides such as saccharose or maltose, molasses, corn starch, oils and fats, while examples of inorganic salts are the customary soluble salts capable of yielding sodium, potassium, iron, zinc, cobalt, magnesium, calcium, ammonium, chloride, carbonate, sulfate, phosphate, nitrate and the like ions.

The reaction course can be monitored by analytical techniques as known in the art, e.g. by HPLC or TLC, by following the disappearance of the starting material and/or the appearance of final products. Usefully, also the appearance/disappearance of intermediates can be monitored with the same techniques.

Fermentation parameters can be conveniently monitored by an automated system controlled by a computer.

The pregneno-oxazolines of formula I are recovered from the filtered fermentation broth as known in the art (see for instance U.S. 2837464 and GB-B- 1205026).

The mycelium and the filtered fermentation broth may be extracted separately with a suitable organic solvent such as chloroform, dichloroethane, benzene, toluene, ethyl acetate and the like.

The extract can be then pooled, partially concentrated and optionally decolorized on carbon and then concentrated to a small volume. Precipitation with a precipitating agent such as an ether gives the compounds of formula I.

The overall yields of this transformation are generally between 50 and 80%.

The compound of formula I then can be used as such or preferably transformed in the above mentioned therapeutically active compounds according to procedures known per se in the art.

The following example further illustrates the invention, and, therefore, cannot be construed as limiting it.

Example 1:

Sequential growth of C. lunata and A. simplex

I) Slant media

Sabouraud medium (for C. lunata)
Antibiotic Agar No. 1 (for A. simplex)

II) Vegetative and pre-culture media

a) for C. lunata

| | |
|---|---|
| Soybean meal | 13 g/l |
| $KH_2PO_4$ | 5 g/l |
| Dextrose | 10 g/l |
| Peptone | 5 g/l |

pH adjusted to 6.5-7.5 before autoclaving

b) for A. simplex

| | |
|---|---|
| Dextrose | 1.0 g/l |
| Soybean meal | 5.0 g/l |
| Peptone | 5.0 g/l |
| Basamin Busch | 3.0 g/l |
| $KH_2PO_4$ | 5.0 g/l |
| NaCl | 5.0 g/l |
| Silicone | 0.1 ml/l |

pH adjusted to 6.5-7.5 before autoclaving.

III) Fermentation media

A fermentation medium having the same composition of the pre-culture medium for C. lunata reported above.

IV) Fermentation procedure

The slants are used to separately inoculate 500 ml flasks which are cultured at about 28 °C for about 12-24 h (C. lunata) or 18-36 h (A. simplex) in the presence of 100 ml of the vegetative media indicated above. These inocula are used in the procedure described below:

Aliquots (about 1 to 5%) of the culture of C. lunata obtained above are transferred in a 8 liter fermentor containing the above reported fermentation medium and cultivated for about 24 h at 29-32 °C.

Then 4 g of 2′-methyl-4-pregnen-21-ol-[-17alpha,16alpha-d]-oxazolinyl-3,20-dione are added and the fermentation is continued until about 36-72 h from the inoculum.

Afterwards, the 18-36 h culture of A. simplex is added thereto and the fermentation is continued for further 40-55 h.

The reaction course is monitored as known in the art by TLC or preferably HPLC by following the disappearance of the starting material and/or appearance of the final product. As a further control, the appearance/disappearance of intermediates can also be followed. HPLC conversion yield: 70-75%.

Example 2:

Recovery

After 40-55 h from the addition of A. simplex, the transformation can be generally considered as completed and the fermentation can be worked up to isolate the desired compound of formula I.

The fermentation mixture is separated by filtration, the mycelium is repeatedly washed with chloroform and the filtrate is extracted with chloroform (3 x 1 l). The combined chloroform washing and extracts are partially concentrated under reduced pressure and decolorized with charcoal. Then they are concentrated to an oily residue. On adding petroleum ether, a precipitate forms which is washed with ether (3 times) and collected by filtration giving 3 g of 2′-methyl-1,4-pregnadien-21-ol-[17alpha,16alpha-d]-oxazolinyl-3,20-dione, that has the following formula:

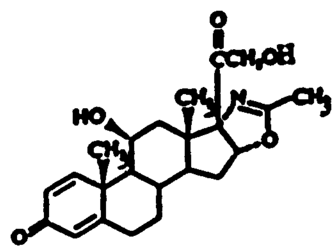

By repeating the above procedure, but using 2′-methyl-4-pregnen-21-ol-[17alpha,16alpha-d]-oxazolinyl-3,20-dione, 21-acetate instead of 2′-methyl-4-pregnen-21-ol-[17alpha,16alpha-d]-oxazolinyl-3,20-dione, the above reported 2′-methyl-1,4-pregnadien-21-ol-[17alpha,16alpha-d]-oxazolinyl-3,20-dione, is obtained substantially with the same yields.

By treating this compound with acetic anhydride in pyridine, Deflazacort is obtained in almost quantitative yields, while by treating it with succinic anhydride in dry pyridine, Deflazacort hemisuccinate is obtained.

**Claims**

**1.** A process for preparing a pregneno-oxazoline of formula I

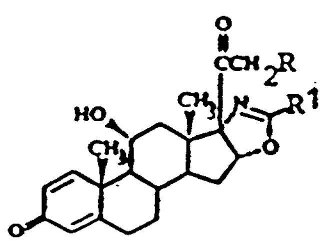

I

wherein

$R^1$ represents hydrogen, ($C_1$-$C_8$)alkyl or phenyl and

R represents hydrogen or hydroxy, which comprises contacting a 2'-substituted-4-pregnen-21-ol-[17alpha,16alpha-d]-oxazolinyl-3,20-dione of formula II

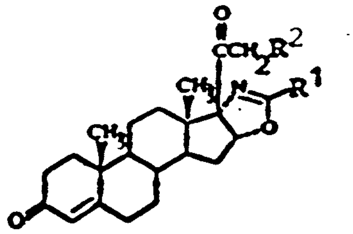

II

wherein

$R^2$ represents hydrogen or acyloxy and $R^1$ is as defined above, with a growing culture of a Curvularia strain and subsequently adding to this mixture a growing culture of an Arthrobacter strain.

**2.** A process according to claim 1 which comprises: adding a compound of formula II to a culture of a Curvularia strain of 12-24 h and then adding a 12-36 h culture of an Arthrobacter strain after 36-96 h from the inoculum of the Curvularia culture.

**3.** A process according to claim 1, or 2 wherein the Curvularia strain is Curvularia lunata NRRL 2380.

**4.** A process according to claim 1, 2 or 3 wherein the Arthrobacter strain is Arthrobacter simplex ATCC 6946.

**5.** A process according to claim 1,2, 3 or 4 wherein $R^2$ in formula II represents hydrogen or acetyloxy.

**6.** A process according to claim 1, 2, 3, 4 or 5 wherein the culture of Curvularia is a culture of 12-24 h.

**7.** A process according to claim 1, 2, 3, 4, 5 or 6 wherein the culture of Arthrobacter is a culture of 12-36 h.

**8.** A process according to claim 1, 2, 3, 4, 5, 6 or 7 wherein the Arthrobacter culture is added to the culture of Curvularia after 24-72 h from the addition of the compound of formula II.

**9.** A process according to claim 1, 2, 3, 4, 5, 6, 7 or 8 wherein the reaction temperature is between 27 °C and 35 °C.

**10.** A process according to claim 1, 2, 3, 4, 5, 6, 7, 8 or 9 wherein the pH of the fermentation medium is adjusted to 6.0-7.5.

**11.** A process according to any one of claims 1 to 10 for preparing 2'-methyl-1,4-pregnadien-21-ol--[17alpha,16alpha-d]-oxazolinyl-3,20-dione, having the following formula:

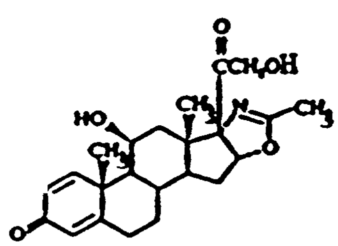

## Patentansprüche

1. Verfahren zur Herstellung eines Pregnen-Oxazolins der Formel I

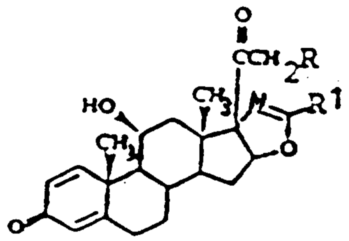

(I)

in der
$R^1$ ein Wasserstoffatom, einen ($C_1$-$C_8$)Alkylrest oder eine Phenylgruppe darstellt und
R ein Wasserstoffatom oder eine Hydroxylgruppe bedeutet, umfassend das Inkontaktbringen eines 2'-substituierten-4-Pregnen-21-ol-[17α,16α-d]-oxazolinyl-3,20-dions der Formel II

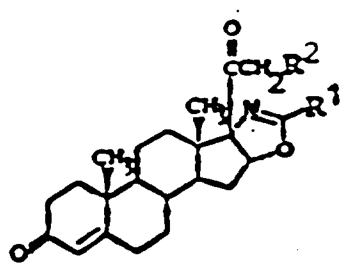

(II)

in der
$R^2$ ein Wasserstoffatom oder einen Acyloxyrest darstellt und $R^1$ die vorstehend angegebene Bedeutung hat, mit einer wachsenden Kultur eines Curvularia-Stamms und das anschließende Hinzufügen einer wachsenden Kultur eines Arthrobacter-Stamms zu diesem Gemisch.

2. Verfahren nach Anspruch 1, umfassend die Zugabe einer Verbindung der Formel II zu einer Kultur eines 12 bis 24 Stunden gewachsenen Curvularia-Stamms und dann Zugabe einer 12 bis 36 Stunden gewachsenen Kultur eines Arthrobacter-Stamms nach 36 bis 96 Stunden von dem Inoculum der Curvularia-Kultur.

3. Verfahren nach Anspruch 1 oder 2, wobei der Curvularia-Stamm Curvularia lunata NRRL 2380 ist.

4. Verfahren nach Anspruch 1, 2 oder 3, wobei der Arthrobacter-Stamm Arthrobacter simplex ATCC 6946 ist.

5. Verfahren nach Anspruch 1, 2, 3 oder 4, wobei $R^2$ in Formel II ein Wasserstoffatom oder einen Acetyloxyrest darstellt.

6. Verfahren nach Anspruch 1, 2, 3, 4 oder 5, wobei die Kultur von Curvularia eine Kultur von 12 bis 24 Stunden ist.

7. Verfahren nach Anspruch 1, 2, 3, 4, 5 oder 6, wobei die Kultur von Arthrobacter eine 12 bis 36 Stunden gewachsene Kultur ist.

8. Verfahren nach Anspruch 1, 2, 3, 4, 5, 6 oder 7, wobei die Arthrobacter-Kultur 24 bis 72 Stunden nach der Zugabe der Verbindung der Formel II zu der Curvularia-Kultur gegeben wird.

**9.** Verfahren nach Anspruch 1, 2, 3, 4, 5, 6, 7 oder 8, wobei die Reaktionstemperatur zwischen 27 ° C und 35 ° C liegt.

**10.** Verfahren nach Anspruch 1, 2, 3, 4, 5, 6, 7, 8 oder 9, wobei der pH-Wert des Züchtungsmediums auf 6,0 bis 7,5 eingestellt wird.

**11.** Verfahren nach einem der Ansprüche 1 bis 10 zur Herstellung von 2'-Methyl-1,4-pregnadien-21-ol-[17α,16α-d]-oxa-zolinyl-3,20-dion mit der folgenden Formel:

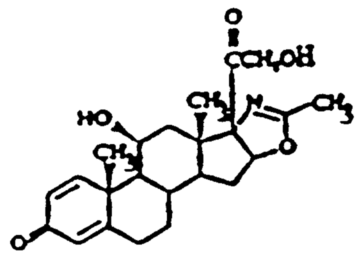

## Revendications

**1.** Procédé de préparation d'une prégnéno-oxazoline répondant à la formule I

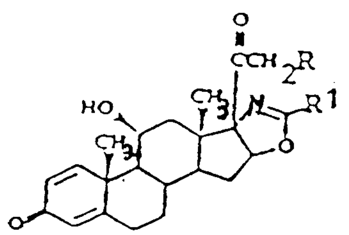

I

dans laquelle
$R^1$ représente un atome d'hydrogène, un groupe alkyle en $C_1$ -$C_8$ ou phényle et
R représente un atome d'hydrogène ou un groupe hydroxy,
qui comprend le fait de mettre en contact une 4-prégnén-21-ol-[17alpha,16alpha-d ]-oxazolinyl-3,20-dione 2'-substituée répondant à la formule II

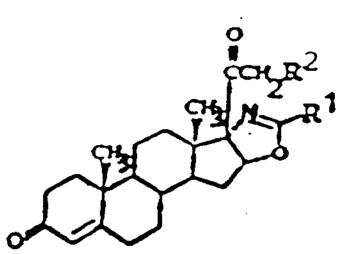

II

dans laquelle
$R^2$ représente un atome d'hydrogène ou un groupe acyloxy et $R^1$ est tel que défini ci-dessus, avec une culture en cours de développement d'une souche de Curvularia, puis d'ajouter à ce mélange une culture en cours de développement d'une souche d'Arthrobacter.

**2.** Procédé selon la revendication 1 qui comprend le fait d'ajouter un composé répondant à la formule II à une culture d'une souche de Curvularia de 12 à 24 heures, puis d'ajouter une culture de 12 à 36

heures d'une souche d'Arthrobacter 36 à 96 heures après l'inoculation de la culture de Curvularia.

3. Procédé selon la revendication 1 ou 2, dans lequel la souche Curvularia est Curvularia lunata NRRL 2380.

4. Procédé selon la revendication 1, 2 ou 3, dans lequel la souche d'Arthrobacter est Arthrobacter simplex ATCC 6946.

5. Procédé selon la revendication 1, 2, 3 ou 4, dans lequel $R^2$ de la formule II représente un atome d'hydrogène ou un groupe acétyloxy.

6. Procédé selon la revendication 1, 2, 3, 4 ou 5, dans lequel la culture de Curvularia est une culture de 12 à 24 heures.

7. Procédé selon la revendication 1, 2, 3, 4, 5 ou 6, dans lequel la culture d'Arthrobacter est une culture de 12 à 36 heures.

8. Procédé selon la revendication 1, 2, 3, 4, 5, 6 ou 7, dans lequel la culture d'Arthrobacter est ajoutée à la culture de Curvularia 24 à 72 heures après l'addition du composé répondant à la formule II.

9. Procédé selon la revendication 1, 2, 3, 4, 5, 6, 7 ou 8, dans lequel la température de réaction est comprise entre 27 ° C et 35 ° C.

10. Procédé selon la revendication 1, 2, 3, 4, 5, 6, 7, 8 ou 9, dans lequel le pH du milieu de fermentation est ajusté à 6,0-7,5.

11. Procédé selon l'une quelconque des revendications 1 à 10 pour la préparation de la 2'-méthyl-1,4-prégnadién-21-ol-[17alpha,16alpha-d]-oxazolinyl-3,20-dione répondant à la formule suivante :